# EUROPEAN PATENT SPECIFICATION

(11) **EP 1 873 151 B1**
(45) Date of publication and mention of the grant of the patent: **10.09.2008**
(21) Application number: 06013133.1
(22) Date of filing: 26.06.2006
(51) Int. Cl.: C07D 403/12

(54) **Improved process for producing moxonidine**
Verbessertes Verfahren zur Herstellung von Moxonidine
Procédé amélioré pour la production de la moxonidine

(43) Date of publication of application: 02.01.2008
(60) Divisional application: 08012788.9
(73) Proprietor: CHEMAGIS LTD., 51200 Bnei-Brak (IL)
(72) Inventor: Naddaka, Vladimir, 71338 Lod (IL); Klopfer, Eyal, 64955 Tel Aviv (IL); Saeed, Shady, 33266 Haifa (IL); Davidi, Guy, 40500 Even-Yehuda (IL); Ostrovsky, Elena, 75233 Rishon-LeZion (IL); Arad, Oded, 76303 Rechovot (IL); Kaspi, Joseph, 53201 Givatayim (IL)
(74) Representative: Becker Kurig Straus

(56) References cited:
- WO-A-20/04037795
- CZ-A- 294 649
- DE-A1- 2 849 537
- DE-A1- 2 937 023
- US-A- 4 323 570
- DATABASE CAPLUS [Online] CHEMICAL ABSTRACTS SERVICE, COLUMBUS, OHIO, US; 22 April 2001 (2001-04-22), XP002409664 retrieved from STN Database accession no. 48:77653
- MINEO SANEYOSHI, SHIN'ICHI WATANABE: CHEMICAL AND PHARMACEUTICAL BULLETIN, vol. 36, no. 7, 1988, pages 2673-2673, XP001249009

## Description

### FIELD OF THE INVENTION

The present invention relates to the field of organic chemistry and more particularly to improved process for producing Moxonidine.

### BACKGROUND OF THE INVENTION

Moxonidine (4-chloro-5-(imidazoline-2-ylamino)-6-methoxy-2-methylpyrimidine), has the structural formula (I) below and is used as an antihypertensive drug.

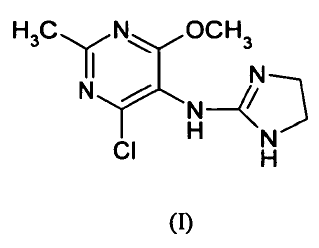

Moxonidine was approved for use in Germany in 1991 and is currently commercially available in Europe, e.g., in Germany, Austria and the UK.

U.S. patent No. 4,323,570 (hereinafter the '570 patent) describes a method of preparing Moxonidine (I) by reacting 4,6-dichloro-2-methyl-5-(1-acetyl-2-imidazolin-2-yl)-aminopyrimidine (hereinafter DMAIA) of formula (II) with about 2 equivalents of sodium methoxide in methanol under reflux, as depicted in Scheme 1.

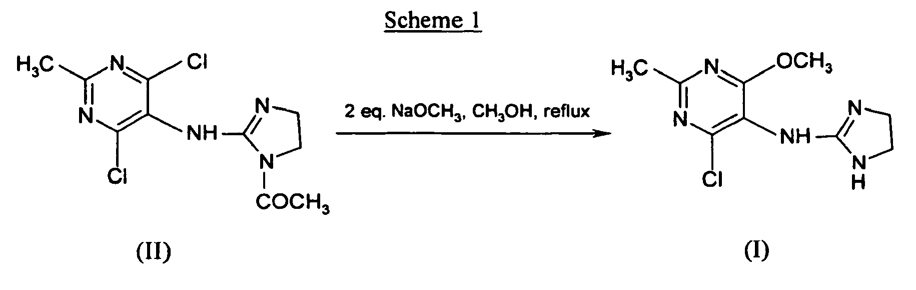

According to example 3 of the '570 patent, Moxonidine is obtained by crystallization from nitromethane.

Czech patent No. 294649 (hereinafter the '649 patent) describes a method of preparing Moxonidine from DMAIA by methanolysis reaction using alkali metal carbonates e.g., potassium carbonate at 47-52°C or sodium bicarbonate at 65°C.

Boiling sodium methoxide is highly corrosive and toxic and therefore there is a need in the art for an improved process for preparing Moxonidine that will use less toxic and corrosive reagents, e.g., sodium hydroxide and potassium hydroxide at a considerably lower concentration (e.g., 1 mole equivalent instead of 2 mole equivalents), and using lower reaction temperatures.

### SUMMARY OF THE INVENTION

The inventors of the present invention have discovered that it is not needed to use sodium methoxide for converting 4,6-dichloro-2-methyl-5-(1-acetyl-2-imidazolin-2-yl)-aminopyrimidine (DMAIA) into Moxonidine as other more user-friendly bases, e.g., sodium hydroxide and potassium hydroxide may be used instead in much milder conditions (ambient temperature and relatively low molar excess of the base). Furthermore, the reaction may be carried out at ambient temperature and it was surprisingly discovered that is not necessary to use two-fold excess of the base as about 1.1 molar excess of the base, e.g., sodium hydroxide in relation to DMAIA is sufficient.

The present invention provides an improved process for producing Moxonidine of formula (I) in a high purity and yield, the process comprising:
reacting 4,6-dichloro-2-methyl-5-(1-acetyl-2-imidazolin-2-yl)aminopyrimidine (DMAIA) with a non-methoxide base in methanol;
diluting the reaction mixture with water;
isolating Moxonidine; and
optionally re-crystallizing Moxonidine from an organic solvent,
wherein the amount of the said non-methoxide base is lower than 2 mole equivalents per one mole equivalent of DMAIA and the non-methoxide base is selected from the group consisting of sodium hydroxide, potassium hydroxide, lithium hydroxide, cesium hydroxide, potassium carbonate, sodium carbonate, lithium carbonate, cesium carbonate, potassium bicarbonate, sodium bicarbonate, lithium bicarbonate, cesium bicarbonate, and combinations thereof.

The present invention also provides a more convenient and environmentally friendly process for purifying Moxonidine by crystallization, wherein the solvent used is other than nitromethane, the process comprising:
mixing the crude Moxonidine with an organic solvent;
heating to an elevated temperature;
allowing the mixture to cool sufficiently to enable crystallization; and
isolating the crystals, washing and drying.

The crude Moxonidine is obtained by the process described herein in at least 88% overall yield, preferably in a yield higher than 90.5%. The crystallized Moxonidine is obtained having a purity of at least 98%, preferably having a purity equal to or greater than 99.5%, and more preferably having a purity equal to or greater than 99.8%.

### DESCRIPTION OF THE PREFERRED EMBODIMENTS

The inventors of the present invention have discovered that it is not needed to use sodium methoxide for converting 4,6-dichloro-2-methyl-5-(1-acetyl-2-imidazolin-2-yl)-aminopyrimidine (DMAIA) into Moxonidine as other more user-friendly bases, e.g., sodium hydroxide and potassium hydroxide may be used instead in much milder conditions (ambient temperature and relatively low molar excess of the base). Furthermore, the reaction may be carried out at ambient temperature and it was surprisingly discovered that it is not necessary to use two-fold excess of the base and about 1.1 molar excess of the base, e.g., sodium hydroxide in relation to DMAIA is sufficient.

While searching for an improved process for preparing Moxonidine, the inventors of the present invention have reproduced example 3 of the '570 patent and example 1 of the '649 patent and found that in both cases the crude Moxonidine contained substantial levels of the impurities 4,6-dimethoxy-2-methyl-5-(2-imidazolin-2-yl)-aminopyrimidine of formula (III) and 4,6-dichloro-2-methyl-5-(2-imidazolin-2-yl)-aminopyrimidine of formula (IV), as described in reference examples 1 and 2 respectively.

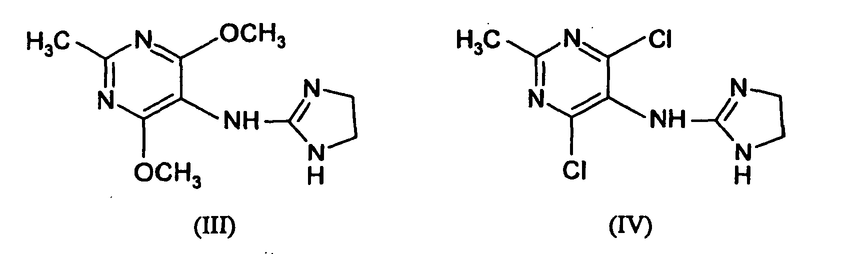

It is apparent to those skilled in the art that purifying crude Moxonidine from these impurities, namely compounds (III) and (IV), may not an easy task to achieve, and the purified Moxonidine is liable to be obtained in a relatively low yield. Therefore, there is a need in the art for an improved process for producing Moxonidine in high purity and yield, wherein the content of the impurities is minimal.

The present invention provides such an improved process for producing Moxonidine as defined above.

The non-methoxide base is selected from sodium hydroxide, potassium hydroxide, lithium hydroxide, cesium hydroxide, potassium carbonate, sodium carbonate, lithium carbonate, cesium carbonate, potassium bicarbonate, sodium bicarbonate, lithium bicarbonate, cesium bicarbonate, and a combination thereof.

According to one embodiment of the present invention, methanol is used as a solvent and the volume of methanol is 4-12 ml relative to 1 g of DMAIA, preferably the volume of methanol is 8 ml relative to 1 g of DMAIA.

In the present invention, a non-methoxide base is used in the reaction and the amount of this base is lower than 2 mole equivalents per one mole equivalent of DMAIA.

According to one aspect of this embodiment of the present invention, the amount of the non-methoxide base, e.g., potassium carbonate is between about 0.6 - 1.0 molar equivalents relative to one mole of DMAIA.

According to another aspect of this embodiment of the present invention, the amount of the non-methoxide base, e.g., sodium hydroxide is between about 1.1-1.5 equivalents relative to one mole of DMAIA.

According to yet another aspect of this embodiment of the present invention, the amount of the non-methoxide base, e.g., potassium hydroxide is between about 1.1-1.7 equivalents relative to one mole of DMAIA.

According to yet another aspect of this embodiment of the present invention, the amount of the non-methoxide base, e.g., sodium bicarbonate is between about 1.0-1.7 equivalents relative to one mole of DMAIA.

According to yet another aspect of this embodiment of the present invention, the amount of the non-methoxide base, e.g., potassium bicarbonate is about 1.5 equivalents relative to one mole of DMAIA.

According to yet another aspect of this embodiment of the present invention, a small amount of water may be added to the mixture containing sodium bicarbonate or potassium bicarbonate e.g., 1 ml per 20 ml of the solvent.

According to yet another aspect of this embodiment of the present invention, the reaction may be carried out using a combination of bases, e.g. potassium hydroxide and potassium carbonate.

According to yet another embodiment of the present invention, the reaction may be carried out at any temperature in the range of 0°C and reflux. Preferably, the temperature for carrying out the reaction is between ambient temperature and 40-50°C, and more preferably the temperature for carrying out the reaction is ambient temperature.

According to yet another embodiment of the present invention, the reaction may be stopped after most of the unwanted intermediate 4,6-dichloro-2-methyl-5-(2-imidazolin-2-yl)-aminopyrimidine of formula (IV) has disappeared, as monitored by HPLC.

According to yet another embodiment of the present invention, after reaction completion, water is added to the reaction mixture and crude Moxonidine is isolated from the resulting suspension by filtration. The crude Moxonidine is then washed with water and with 2-propanol and dried.

The present invention also provides a process for purifying Moxonidine by crystallization, wherein the solvent used is other than nitromethane, the process comprising:
mixing the crude Moxonidine with a solvent;
heating to an elevated temperature;
allowing the mixture to cool sufficiently to enable crystallization; and
isolating the crystals, washing and drying.

Typically, the crystallization solvent is a class 3 solvent, e.g., acetone, 1-propanol, 2-propanol, 1-butanol, 2-butanol, dimethyl sulfoxide (DMSO), and a mixture thereof.

The crude Moxonidine is obtained by the process described herein in at least 88% overall yield, preferably in a yield higher than 90.5%. The crystallized Moxonidine is obtained
having a purity of at least 98%, preferably having a purity equal to or greater than 99.5%, and more preferably having a purity equal to or greater than 99.8%.

### Examples

HPLC measurements of Moxonidine samples were performed using HPLC system, equipped with Phenomenex Luna 5µ C8(2) (4.6 x 250 mm) column, and a UV detector operated on 230 nm. Analyses were performed using the following mobile phase, at flow rate of 1.2 ml/minute. Eluent A: 10 mM pentanesulfonic acid, pH = 3.0 with H₂SO₄. Eluent B: acetonitrile. Gradient (A/B, v/v): 0 min (94/6), 4 min (94/6), 20 min (64/36), 50 min (64/36).

### Reference Example 1 - Preparation of Moxonidine by reaction of 4,6-dichloro-2-methyl-5-(1-acetyl-2-imidazolin-2-yl)-aminopyrimidine (DMAIA) with 2.02 equivalents of sodium methoxide at boiling temperature

4,6-dichloro-2-methyl-5-(1-acetyl-2-imidazolin-2-yl)-aminopyrimidine (10 g, 0.0347 mol) was mixed with a solution of sodium methoxide (3.78 g, 0.07 mol, 2.02 equiv.) in 35 ml of methanol and boiled for 2 hours. Then, water (100 ml) was added and the reaction mixture was cooled to ambient temperature. A colorless product was collected by filtration, washed with water and dried at 50° C overnight to yield crude Moxonidine containing 6.5% of impurity (III) and 0.61 % of impurity (IV).

### Reference Example 2 - Preparation of Moxonidine by reaction of DMAIA with 2 molar equivalents of potassium carbonate at 47-52° C.

Potassium carbonate (9.6 g, 0.0694 mol, 2 molar equiv.) was added to a suspension of DMAIA (10 g, 0.0347 mol) in methanol (80 ml) and the mixture was heated at 47-52° C for 5 hours. Then, the reaction mixture was cooled to ambient temperature and acetic acid (10 ml) and water (70 ml) were added. After stirring for half an hour, 25% solution of ammonium hydroxide (10 ml) was added and the mixture was stirred for one hour. A colorless precipitate was collected by filtration, washed with water and dried at 50° C overnight to yield crude Moxonidine containing 0.07% of impurity (III) and 0.40% of impurity (IV).

### Example 1 (not according to the invention) - Preparation of Moxonidine by reaction of DMAIA with 1.1 equivalents of sodium methoxide at ambient temperature

Sodium methoxide (25% solution in methanol, 7.7 ml, 0.0382 mol, 1.1 equiv.) was added at ambient temperature to a suspension of DMAIA (10 g, 0.0347 mol) in methanol (50 ml) and the reaction mixture was stirred at ambient temperature for 24 hours. Then, water (100 ml) was added and the reaction mixture was cooled to ambient temperature. A colorless product was collected by filtration, washed with water (3 x10 ml) and 2-propanol (3 x10 ml) and dried at 50° C overnight to give 7.4 g of crude Moxonidine in 88.3% yield, having a purity of 99.17% (by HPLC), containing 0.04% of impurity (III) and 0.71% of impurity (IV).

### Examples 2 - 9 (not according to the invention)

The same reaction, which is provided in example 1, was carried out using sodium methoxide in methanol at different reaction conditions, or alternatively using other solvents than methanol, as depicted in Table 1

**Table 1 - Preparation of crude Moxonidine by reaction of DMAIA with sodium methoxide in methanol and other solvents**

| Ex No | DMAIA, g (mol) | Solvent, (ml) | Base, (mol, equiv.) | Temp., °C | Time, h | Crude yield g (%) | Purity, % | % of (III) | % of (IV) |
|---|---|---|---|---|---|---|---|---|---|
| 2 | 10(0.035) | MeOH | CH₃ONa | 25 | 24 | 7.4 | 98.27 | 0.02 | 1.56 |
| | | (50) | (0.035, 1.0) | | | (88.3) | | | |
| 3 | 10(0.035) | MeOH | CH₃ONa | 25 | 18 | 7.25 | 99.5 | 0.06 | 0.44 |
| | | (100) | (0.059,1.7) | | | (86.5) | | | |
| 4 | 10(0.035) | MeOH | CH₃ONa | 65 | 1.5 | 7.4 | 96.74 | 0.05 | 3.21 |
| | | (35) | (0.035,1.0) | | | (88.3) | | | |
| 5 | 5 (0.017) | MeOH | CH₃ONa | 0 | 48 | 3.25 | 98.55 | - | 1.45 |
| | | (50) | (0.016,0.9) | | | (77.6) | | | |
| 6 | 5 (0.017) | MeOH | CH₃ONa | 0 | 48 | 3.55 | 99.35 | 0.09 | 0.48 |
| | | (30) | (0.017, 1.0) | | | (84.7) | | | |
| 7 | 5 (0.017) | THF | CH₃ONa | 25 | 30 | 3.2 | 92.48 | 0.10 | 7.44 |
| | | (50) | (0.019, 1.1) | | | (76.4) | | | |
| 8 | 5 (0.017) | Toluene | CH₃ONa | 25 | 30 | 2.8 | 94.76 | 0.20 | 4.86 |
| | | (50) | (0.019, 1.1) | | | (66.8) | | | |
| 9 | 5 (0.017) | DMSO | CH₃ONa | 25 | 30 | 3.2 | 98.23 | 0.44 | - |
| | | (50) | (0.019, 1.1) | | | (76.4) | | | |

### Example 10 - Preparation of Moxonidine by reaction of DMAIA with 1.0 molar equivalent of potassium carbonate

A mixture of DMAIA (5 g, 0.0174 mol) and potassium carbonate (2.4 g, 0.0174 mol, 1.0 molar equiv.) in methanol (40 ml) was heated at 45-50° C for 18 hours. Then, the reaction mixture was cooled to ambient temperature and acetic acid (4 ml) and water (35 ml) were added. After stirring for half an hour, 25% solution of ammonium hydroxide (4 ml) was added and the mixture was stirred for one hour. A colorless precipitate was collected by filtration, washed with water and dried at 50° C overnight to give 3.8 g of crude Moxonidine in 90.7% yield, having a purity of 99.23% (by HPLC), containing 0.10% of impurity (III) and 0.67% of impurity (IV).

### Examples 11-12

The same reaction, which is provided in example 10, was carried out using potassium carbonate in methanol at different reaction conditions, as depicted in Table 2.

**Table 2 - Preparation of crude Moxonidine by reaction of DMAIA with potassium carbonate**

| Ex No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 11 | 5 (0.017) | MeOH (40) | K₂CO₃ (0.017, 1.0) | 65 | 3 | 3.8 (90.7) | 99.0 | 1.0 | - |
| 12 | 5 (0.017) | MeOH (40) | K₂CO₃ (0.01,0.6) | 65 | 4 | 3.4 (81.1) | 99.33 | 0.6 | 0.07 |

### Example 13 - Preparation of Moxonidine by reaction of DMAIA with 1.5 equivalents of 47% aqueous solution of sodium hydroxide

A mixture of DMAIA (5 g, 0.0174 mol) and 47% aqueous solution of sodium hydroxide (1.25 g, 0.026 mol, 1.5 equiv.) in methanol (40 ml) was stirred at 40-50° C for 10 hours. Then, the reaction mixture was cooled to ambient temperature and water (50 ml) was added. After stirring for one hour, a colorless precipitate was collected by filtration, washed with water (3 ×10 ml) and 2-propanol (3 ×10 ml) and dried at 50° C overnight to yield 3.7 g of crude Moxonidine in 88.3% yield, having a purity of 99.52% (by HPLC), containing 0.04% of impurity (III) and 0.44% of impurity (IV).

### Examples 14-16

The same reaction, which is provided in example 13, was carried out using sodium hydroxide solution in methanol at different reaction conditions, as depicted in Table 3.

**Table 3 - Preparation of crude Moxonidine by reaction of DMAIA with sodium hydroxide solution**

| Ex No | DMAIA, g (mol) | Solvent, (ml) | Base, (mol, equiv.) | Temp., °C | Time, h | Crude yield g (%) | Purity, % | % of (III) | % of (IV) |
|---|---|---|---|---|---|---|---|---|---|
| 14 | 5 (0.017) | MeOH (40) | 47% aq. NaOH sol. (0.019, 1.1) | 25 | 24 | 3.7 (88.3) | 98.54 | - | 1.46 |
| 15 | 5 (0.017) | MeOH (40) | 47% aq. NaOH sol. (0.023, 1.3) | 55-60 | 5 | 3.7 (88.3) | 99.47 | 0.08 | 0.45 |
| 16 | 5 (0.017) | MeOH (40) | 47% aq. NaOH sol. (0.019, 1.1) | 65 | 2 | 3.4 (81.1) | 98.86 | 0.13 | 1.01 |

### Example 17 - Preparation of Moxonidine by reaction of DMAIA with 1.3 equivalents of 85% potassium hydroxide powder at ambient temperature

A mixture of DMAIA (5 g, 0.0174 mol) and 85% potassium hydroxide powder (1.5 g, 0.0226 mol, 1.3 equiv.) in methanol (40 ml) was stirred at ambient temperature for 24 hours. Then, water (50 ml) was added and the mixture was stirred for one hour. A colorless precipitate was collected by filtration, washed with water (3 x10 ml) and 2-propanol (3 x10 ml) and dried at 50° C overnight to give 3.7 g of crude Moxonidine in 88.3% yield, having a purity of 99.49% (by HPLC), containing 0.01% of impurity (III) and 0.50% of impurity (IV).

### Examples 18 -19

The same reaction, which is provided in example 17, was carried out using 85% potassium hydroxide powder in methanol at different reaction conditions, as depicted in Table 4.

**Table 4 - Preparation of crude Moxonidine by reaction of DMAIA with solid potassium hydroxide**

| No | DMAIA, g (mol) | Solvent, (ml) | Base, (mol, equiv.) | Temp., °C | Time, *h* | Crude yield g (%) | Purity, % | % of (III) | % of (IV) |
|---|---|---|---|---|---|---|---|---|---|
| 18 | 5 (0.017) | MeOH (40) | 85% KOH powder (0.019, 1.1) | 25 | 30 | 3.5 (83.5) | 98.21 | 0.02 | 1.77 |
| 19 | 5 (0.017) | MeOH (40) | 85% KOH powder (0.026, 1.5) | 25 | 12 | 3.7 (88.3) | 99.50 | - | 0.50 |

### Example 20 - Preparation of Moxonidine by reaction of DMAIA with 45% aqueous potassium hydroxide solution at ambient temperature

A mixture of DMAIA (5 g, 0.0174 mol) and 45% aqueous potassium hydroxide solution (1.8 g, 0.029 mol, 1.7 equiv.) in methanol (40 ml) was stirred at ambient temperature for 10 hours. Then, water (50 ml) was added and the mixture was stirred for one hour. A colorless precipitate was collected by filtration, washed with water (3 x10 ml) and 2-propanol (3 x10 ml) and dried at 50° C overnight to give 3.7 g of crude Moxonidine in 88.3% yield, having a purity of 99.45% (by HPLC), containing 0.03% of impurity (III) and 0.52% of impurity (IV).

### Examples 21 -25

The same reaction, which is provided in example 20, was carried out using aqueous potassium hydroxide in methanol at different reaction conditions, as depicted in Table 5.

**Table 5 - Preparation of crude Moxonidine by reaction of DMAIA with aqueous potassium hydroxide**

| Ex. No | DMAIA, g (mol) | Solvent, (ml) | Base, (mol, equiv.) | Temp., °C | Time, *h* | Crude Yield g(%) | Purity, % | % of (III) | % of (IV) |
|---|---|---|---|---|---|---|---|---|---|
| 21 | 5 (0.017) | MeOH (40) | 45% aq. KOH sol. (0.023, 1.3) | 65 | 3 | 3.6 (85.9) | 99.34 | 0.19 | 0.47 |
| 22 | 5 (0.017) | MeOH (40) | 45% aq. KOH sol. (0.019, 1.1) | 65 | 2 | 3.4 (81.1) | 98.28 | 0.20 | 1.52 |
| 23 | 5 (0.017) | MeOH (40) | 45% aq. KOH sol. (0.023, 1.3) | 25 | 11 | 3.6 (85.9) | 98.71 | 0.01 | 1.28 |
| 24 | 5 (0.017) | MeOH (40) | 45% aq. KOH sol. | 25 | 10 | 3.6 (85.9) | 99.04 | 0.02 | 0.93 |
| 25 | 5 (0.017) | MeOH (40) | 45% aq. KOH sol. (0.029, 1.7) | 25 | 10 | 3.7 (88.3) | 99.17 | 0.02 | 0.81 |

### Examples 26 -29

The same reaction, which is provided in example 17, was carried out using sodium bicarbonate powder in methanol, optionally with addition of small volume of water (example 29), at different reaction conditions, as depicted in Table 6.

**Table 6 - Preparation of crude Moxonidine by reaction of DMAIA with sodium bicarbonate**

| Ex. No | DMAIA, g (mol) | Solvent, (ml) | Base, (mol, equiv.) | Temp., °C | Time, h | Crude Yield g (%) | Purity, % | % of (III) | % of (IV) |
|---|---|---|---|---|---|---|---|---|---|
| 26 | 5 (0.017) | MeOH (40) | NaHCO₃ (0.017, 1.0) | 65 | 14.5 | 3.6 (85.9) | 98.71 | 0.56 | 0.65 |
| 27 | 5 (0.017) | MeOH (40) | NaHCO₃ (0.023, 1.3) | 65 | 17.5 | 3.7 (88.3) | 97.82 | 1.32 | 0.86 |
| 28 | 5 (0.017) | MeOH (40) | NaHCO₃ (0.026, 1.5) | 40-50 | 24 | 2.9 (71.1) | 97.85 | 1.87 | 0.14 |
| 29 | 5 (0.017) | MeOH (40), water (2) | NaHCO₃ (0.029, 1.7) | 65 | 6 | 3.7 (88.3) | 98.59 | 0.28 | 1.13 |

### Examples 30 -31

The same reaction, which is provided in example 17, was carried out using potassium bicarbonate powder in methanol, optionally with addition of small volume of water (example 31), at different reaction conditions, as depicted in Table 7.

**Table 7 - Preparation of crude Moxonidine by reaction of DMAIA with potassium bicarbonate**

| Ex. No | DMAIA, g (mol) | Solvent, (ml) | Base, (mol, equiv.) | Temp., °C | Time, h | Crude Yield g (%) | Purity, % | % of (III) | % of (IV) |
|---|---|---|---|---|---|---|---|---|---|
| 30 | 5 (0.017) | MeOH (40) | KHCO₃ (0.026, 1.5) | 65 | 9 | 3.6 (85.9) | 99.39 | 0.53 | 0.08 |
| 31 | 5 (0.017) | MeOH (40), water (2) | KHCO₃ (0.026, 1.5) | 65 | 10 | 3.7 (88.3) | 99.20 | 0.35 | 0.45 |

### Example 32 - Preparation of Moxonidine by reaction of DMAIA with a mixture of aqueous potassium hydroxide and solid potassium carbonate at ambient temperature

A mixture of DMAIA (5 g, 0.0174 mol) and 45% potassium hydroxide solution (0.023 mol, 1.3 equiv.) and solid potassium carbonate (0.004 mol, 0.2 equiv.) in methanol (40 ml) was stirred at ambient temperature for 16 hours. Then, water (50 ml) was added and the mixture was stirred for one hour. A colorless precipitate was collected by filtration, washed with water (3 x10 ml) and 2-propanol (3 x10 ml) and dried at 50° C overnight to yield 3.6 g of crude Moxonidine in 85.9% yield, having a purity of 99.07% (by HPLC), containing 0.03% of impurity (III) and 0.90% of impurity (IV).

### Example 33 - Crystallization of Moxonidine from DMSO

Crude Moxonidine (2.0 g), having a purity of 99.12% and containing 0.03% of impurity (III) and 0.85% of impurity (IV), was mixed with DMSO (10 ml) and heated to a temperature of about 90°C to obtain a clear solution. The solution was cooled to ambient temperature and the thus formed crystals were obtained by filtration and washed with cold 2-propanol and dried under reduced pressure. 1.7 g of crystallized Moxonidine were obtained in 85% yield having a purity by HPLC of 99.91 %, and containing 0.02% of impurity (III) and 0.06% of impurity (IV)
Table 8 contains the results of crystallization from different solvents.

**Table 8. Crystallization of crude Moxonidine from different solvents**

| Ex. No. | Solvent | volume, ml | Moxonidine crude, g | Yield, % | Purity, % | % of (III) | % of (IV) |
|---|---|---|---|---|---|---|---|
| 34 | 1-propanol | 48 | 2 | 85 | 99.61 | 0.04 | 0.30 |
| 35 | 2-propanol | 80 | 2 | 80 | 99.35 | 0.03 | 0.62 |
| 36 | 1-butanol | 52 | 2 | 85 | 99.70 | 0.05 | 0.25 |

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention.

## Claims

1. A process for preparing Moxonidine, comprising:
reacting 4,6-dichloro-2-methyl-5-(1-acetyl-2-imidazolin-2-yl)aminopyrimidine (DMAIA) with a non-methoxide base in methanol;
diluting the reaction mixture with water;
isolating Moxonidine; and
optionally re-crystallizing Moxonidine from an organic solvent,
wherein the amount of the said non-methoxide base is lower than 2 mole equivalents per one mole equivalent of DMAIA and it is selected from the group consisting of sodium hydroxide, potassium hydroxide, lithium hydroxide, cesium hydroxide, potassium carbonate, sodium carbonate, lithium carbonate, cesium carbonate, potassium bicarbonate, sodium bicarbonate, lithium bicarbonate, cesium bicarbonate, and combinations thereof.

2. The process of claim 1 wherein the base is sodium hydroxide, potassium hydroxide, potassium carbonate, sodium bicarbonate, potassium bicarbonate, or a combination thereof.

3. The process of claim 1, wherein the reaction is carried out at a temperature range between 0°C and reflux.

4. The process of claim 2, wherein between about 1.1-1.5 equivalents of sodium hydroxide are used with respect to 1 equivalent of DMAIA.

5. The process of claim 2, wherein between about 1.1-1.7 equivalents of potassium hydroxide are used with respect to 1 equivalent of DMAIA and the reaction is carried out at ambient temperature.

6. The process of claim 2, wherein between about 0.6-1.0 equivalents of potassium carbonate are used with respect to 1 equivalent of DMAIA.

7. The process of claim 2, wherein between 1.0-1.7 equivalents of sodium bicarbonate are used with respect to 1 equivalent of DMAIA.

8. The process of claim 2, wherein 1.5 equivalents of potassium bicarbonate are used with respect to 1 equivalent of DMAIA.

9. The process of claim 2, wherein water is added to the mixture containing sodium bicarbonate or potassium bicarbonate.

10. The process of claim 1, wherein water is added to the reaction mixture after reaction completion, and the crude Moxonidine is isolated from the resulting suspension by filtration, washed with water and 2-propanol and dried.

## Patentansprüche

1. Verfahren zur Herstellung von Moxonidin, umfassend:
- Reagieren von 4,6-Dichlor-2-methyl-5-(1-acetyl-2-imidazolin-2-yl)-aminopyrimidin (DMAIA) mit einer Nicht-Methoxid-Base in Methanol;
- Verdünnen des Reaktionsgemisches mit Wasser;
- Isolieren von Moxonidin; und
- optional Rekristallisieren von Moxonidin aus einem organischen Lösungsmittel,
- wobei die Menge der Nicht-Methoxid-Base kleiner als 2 Mol Äquivalente pro ein Mol Äquivalent von DMAIA und aus der Gruppe bestehend aus Natrium-Hydroxid, Kalium-Hydroxid, Lithium-Hydroxid, Cäsium-Hydroxid, Kalium-Carbonat, Natrium-Carbonat, Lithium-Carbonat, Cäsium-Carbonat, Kalium-Bicarbonat, Natrium-Bicarbonat, Lithium-Bicarbonat, Cäsium-Bicarbonat und Kombinationen davon ausgewählt ist.

2. Verfahren nach Anspruch 1, wobei die Base Natrium-Hydroxid, Kalium-Hydroxid, Kalium-Carbonat, Natrium-Bicarbonat, Kalium-Bicarbonat oder eine Kombination davon ist.

3. Verfahren nach Anspruch 1, wobei die Reaktion in einem Temperaturbereich zwischen 0°C und Rückfluss ausgeführt wird.

4. Verfahren nach Anspruch 2, wobei zwischen 1,1-1,5 Äquivalente von Natrium-Hydroxid in Bezug auf 1 Äquivalent DMAIA verwendet werden.

5. Verfahren nach Anspruch 2, wobei zwischen 1,1-1,7 Äquivalente von Kalium-Hydroxid in Bezug auf 1 Äquivalent DMAIA verwendet werden und die Reaktion bei Raumtemperatur ausgeführt werden.

6. Verfahren nach Anspruch 2, wobei zwischen 0,6-1,0 Äquivalente von Kalium-Carbonat in Bezug auf 1 Äquivalent DMAIA verwendet werden.

7. Verfahren nach Anspruch 2, wobei zwischen 1,0-1,7 Äquivalente von Natrium-Bicarbonat in Bezug auf 1 Äquivalent DMAIA verwendet werden.

8. Verfahren nach Anspruch 2, wobei 1,5 Äquivalente von Kalium-Bicarbonat in Bezug auf 1 Äquivalent DMAIA verwendet werden.

9. Verfahren nach Anspruch 2, wobei Wasser zu dem Gemisch, welches Natrium-Bicarbonat oder Kalium-Bicarbonat enthält, hinzugefügt wird.

10. Verfahren nach Anspruch 1, wobei nach Ablauf der Reaktion zu dem Reaktionsgemisch Wasser hinzugefügt wird und das Roh-Moxonidin von der so erhaltenen Suspension isoliert wird durch Filtrieren, Waschen mit Wasser und 2-Propanol und Trocknen.

## Revendications

1. Procédé de préparation de la Moxonidine, comprenant :
la réaction de 4,6-dichloro-2-méthyl-5-(1-acétyl-2-imidazolin-2-yl)amino-pyrimidine (DMAIA) avec une base qui n'est pas un méthoxyde dans du méthanol ;
la dilution du mélange réactionnel avec de l'eau ;
l'isolement de la Moxonidine ; et
éventuellement la recristallisation dans un solvant organique,
dans lequel la quantité de ladite base qui n'est pas un méthoxyde est inférieure à 2 équivalents molaires pour un équivalent molaire de DMAIA et elle est choisie dans le groupe constitué d'hydroxyde de sodium, d'hydroxyde de potassium, d'hydroxyde de lithium, d'hydroxyde de césium, de carbonate de potassium, de carbonate de sodium, de carbonate de lithium, de carbonate de césium, de bicarbonate de potassium, de bicarbonate de sodium, de bicarbonate de lithium, de bicarbonate de césium et de combinaisons de ceux-ci.

2. Procédé selon la revendication 1, dans lequel la base est l'hydroxyde de sodium, l'hydroxyde de potassium, le carbonate de potassium, le bicarbonate de sodium, le bicarbonate de potassium, ou une combinaison de ceux-ci.

3. Procédé selon la revendication 1, dans lequel la réaction est réalisée dans une plage de températures entre 0°C et le reflux.

4. Procédé selon la revendication 2, dans lequel entre environ 1,1 et 1,5 équivalent d'hydroxyde de sodium est utilisé par rapport à 1 équivalent de DMAIA.

5. Procédé selon la revendication 2, dans lequel environ 1,1 et 1,7 équivalent d'hydroxyde de potassium est utilisé par rapport à 1 équivalent de DMAIA et la réaction est réalisée à température ambiante.

6. Procédé selon la revendication 2, dans lequel entre environ 0,6 et 1,0 équivalent de carbonate de potassium est utilisé par rapport à 1 équivalent de DMAIA.

7. Procédé selon la revendication 2, dans lequel entre environ 1,0 et 1,7 équivalent de bicarbonate de sodium est utilisé par rapport à 1 équivalent de DMAIA.

8. Procédé selon la revendication 2, dans lequel environ 1,5 équivalent de bicarbonate de potassium est utilisé par rapport à 1 équivalent de DMAIA.

9. Procédé selon la revendication 2, dans lequel de l'eau est ajoutée au mélange contenant du bicarbonate de sodium ou du bicarbonate de potassium.

10. Procédé selon la revendication 1, dans lequel de l'eau est ajoutée au mélange réactionnel au terme de la réaction, et la Moxonidine brute est isolée de la suspension résultante par filtration, lavée avec de l'eau et du 2-propanol et séchée.
